(19) Europäisches Patentamt
European Patent Office
Office européen des brevets

(11) Numéro de publication : **0 141 755**
**B1**

# (12) FASCICULE DE BREVET EUROPÉEN

(45) Date de publication du fascicule du brevet : **20.01.88**

(51) Int. Cl.[4] : **C 07 D233/40**, D 06 M 13/38

(21) Numéro de dépôt : **84402239.2**

(22) Date de dépôt : **07.11.84**

(54) **Procédé de fabrication d'urées cycliques de structure imidazolidinique et son application notamment dans la fabrication d'apprêts textiles.**

(30) Priorité : **08.11.83 FR 8317702**

(43) Date de publication de la demande :
**15.05.85 Bulletin 85/20**

(45) Mention de la délivrance du brevet :
**20.01.88 Bulletin 88/03**

(84) Etats contractants désignés :
**CH DE FR GB LI**

(56) Documents cités :
**US-A- 3 091 617**
**US-A- 3 260 565**
**Le dossier contient des informations techniques présentées postérieurement au dépôt de la demande et ne figurant pas dans le présent fascicule.**

(73) Titulaire : **SOCIETE FRANCAISE HOECHST Société anonyme dite:**
**3, avenue du Général de Gaulle**
**F-92800 Puteaux (FR)**

(72) Inventeur : **Mabire, Frédéric**
**82, Boulevard Masséna**
**F-75648 Paris Cedex 13 (FR)**
Inventeur : **Blanc, Alain**
**21Bis, rue Galvani**
**F-75017 Paris (FR)**

(74) Mandataire : **Rinuy, Santarelli**
**14, avenue de la Grande Armée**
**F-75017 Paris (FR)**

**EP 0 141 755 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

La présente invention concerne un procédé de préparation d'urées cycliques, de structure imidazolidinique, et son application.

Les urées cycliques de structure imidazolidinique et particulièrement la dihydroxy-4,5 imidazolidinone-2, ci-après désigné DHEU, et la dihydroxy-4,5 diméthyl-1,3 imidazolidinone-2, ci-après désignée $DM_eDHEU$, sont des matières premières couramment employées pour préparer des apprêts textiles destinés aux traitements infroissables irrétrécissables des tissus cellulosiques.

Ces dernières années, ces apprêts ont acquis une importance industrielle considérable, si bien que des recherches se poursuivent constamment pour en obtenir de plus performants, moins coûteux, ne jaunissant pas lors des traitements au chlore, stables aux lavages ménagers, dépourvus d'effets secondaires susceptibles d'affecter les qualités du tissu apprêté. Une solution à ce problème est de pouvoir fabriquer à faibles prix, ces urées cycliques de structure imidazolidinique pures soit en solution aqueuse, soit à l'état cristallisé.

Il est connu d'accéder à ces urées cycliques de structure imidazolidinique par condensation du glyoxal avec une urée de formule générale (I)

RNH—CO—NHR (I)

dans laquelle R représente un atome d'hydrogène ou un radical alkyle en $C_1$-$C_4$.

De telles urées cycliques de formule générale (II)

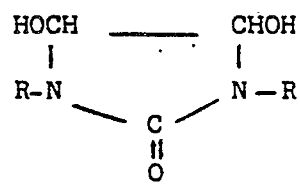

(II)

dans laquelle R a la signification donnée précédemment, telles que la DHEU, la $DM_eDHEU$ sont connues et décrites.

C'est ainsi qu'il est possible d'obtenir selon le brevet des Etats-Unis d'Amérique n° 3.091.617 une dialkyl-1,3 dihydroxy-4,5 imidazolidinone-2 par condensation de glyoxal avec la dialkyl-1,3 urée correspondante en milieu alcoolique et à pH inférieur 4 en présence d'un acide minéral ou organique fort tel que l'acide orthosphosphorique ou d'obtenir selon les exemples du brevet des Etats-Unis d'Amérique n° 3.260.565 des urées cycliques de formule générale (II) par condensation de glyoxal avec l'urée correspondante de formule générale (I) en milieu aqueux et à pH = 7.

Par ailleurs, certains auteurs, ont pu isoler à l'état cristallisé la cis et la trans $DM_eDHEU$ en opérant en milieu alcalin (Sidney L. Vail et al, J. Org. Chem. 1965, 30, 2179-2182). Mais ces urées cycliques sont obtenues à l'état cristallisé avec de faibles rendements même après de longues durées de condensation entre le glyoxal et l'urée substituée ou non.

Ces auteurs ont même pu isoler à l'état cristallisé la cis et la trans $DM_eDHEU$.

Mais ces urées cycliques de formule générale (II) sont obtenues à l'état cristallisé avec de faibles rendements même après de longues durées de condensation entre le glyoxal et l'urée de formule générale (I).

En effet, on sait que la condensation du glyoxal avec l'urée est complexe et que, selon les conditions opératoires, le processus de cyclisation est susceptible de s'arrêter au stade d'addition primaire dihydroxylé cherché (Sidney L. Vail et al., Amer. Dyestuff Reporter, 1961, 550-553 ; brevets US-A-2 731 472 et 3 260 565, N° 3 304 312) ou de se poursuivre soit par une double cyclisation jusqu'au glycolurile : tétrahydroimidazo [4,5-d] imidazole-(1H,3H)dione-2,5 (J. NEMATOLLANI et al, J. Org. Chem., 1963, 28, 2378-2380 ; H. Biltz, Ber., 1907, 40, 4808) soit par une double condensation symétrique avec du glyoxal non transformé jusqu'au bis (dihydroxy-4,5 oxo-2 imidazolidinyl-1)-1,2 dihydroxy-1,2 éthane (E. KANTSCHEV et al, Textilveredlung, 1981, 16, 414-417). D'autres déviations sont également observées comme la formation d'hydantoïne (H. PAULY et al, Ber., 1930, 63, 2063-2069) ou la dégradation du glyoxal selon Cannizzaro.

De plus, les urées cycliques de formule générale (II) présentent une isomérie géométrique cis-trans au niveau des atomes de carbone substitués par les groupements hydroxyle et il est admis, aujourd'hui, que la condensation du glyoxal avec une urée de formule générale (I) n'est pas stéréosélective, et qu'il se forme, tout au moins dans un premier temps, autant d'isomère cis que d'isomère trans, puis que cet équilibre est déplacé vers la forme trans la plus stable (Sidney L. Vail et al, J. Org. Chem., 1965, 30, 2179-2182).

En conséquence, dans les procédés de préparation d'urées cycliques de formule générale (II) par condensation du glyoxal avec l'urée convenablement substituée, le rendement en urée cyclique cherchée, d'une part, et sa propension à cristalliser dans son milieu réactionnel, d'autre part, dépendent

de très nombreux facteurs dont le principal, reconnu comme tel dans l'état de la technique, est le pH de la condensation qui doit être soit compris entre 4 et 8 (J. G. FRICK et al, Ing. and Eng. Chem., Product Research and Development, 1982, 21, 599-600) soit stabilisé à une valeur comprise entre 4 et 6,8 par un tampon en quantité adéquate (brevet US-A-4 295 846).

Or, la Demanderesse a découvert avec étonnement, que l'emploi, en quantité catalytique, d'acide orthophosphorique, dans les procédés connus de préparation d'urées cycliques de formule générale (II) par condensation en milieu aqueux, à pH compris entre 4 et 7 et à une température inférieure à 60 °C, de glyoxal avec une durée convenablement substituée, de formule générale (I) améliore la vitesse de la condensation attendue, réduit de manière significative les vitesses des réactions secondaires parasites et permet d'augmenter les rendements en urée cyclique cherchée pure soit cristallisée, soit brute en solution dans son milieu réactionnel.

La présente invention concerne donc un procédé de préparation d'urées cycliques de formule générale (II) par condensation de glyoxal avec une urée de formule générale (I), en excès ou non, en milieu aqueux et à une température comprise entre 40 °C et 60 °C, caractérisé par le fait qu'il est effectué à un pH compris entre 4 et 7 et en présence d'une quantité catalytique d'acide orthophosphorique.

Selon l'invention, le procédé a tout spécialement pour objet la préparation d'urées cycliques de formule générale (II) dans laquelle R représente un atome d'hydrogène ou un radical méthyle.

La quantité d'acide orthophosphorique utilisée dans le procédé selon l'invention peut varier dans des proportions assez importantes selon les paramètres réactionnels et/ou la nature des réactifs. L'expérience montre que la vitesse réactionnelle est d'autant plus rapide que la concentration en acide orthophosphorique est plus élevée. Toutefois, à partir d'une concentration supérieure à 150 mmoles d'acide orthophosphorique par mole de glyoxal engagé, le rendement en urée cyclique cherchée, isolée à l'état cristallisé n'est plus modifié et a même tendance à diminuer si cette concentration augmente.

En conséquence, un bon compromis est trouvé lorsqu'on met en œuvre le procédé selon l'invention avec de 2 à 150 mmoles d'acide orthophosphorique par mole de glyoxal engagé.

Selon les conditions préférentielles de réalisation de l'invention pour la préparation d'urées cycliques de formule générale (II) dans laquelle R représente un atome d'hydrogène ou un radical méthyle, le procédé ci-dessus décrit, est réalisé en présence de 20 à 60 mmoles d'acide orthophosphorique par mole de glyoxal engagé.

Les études réalisées par suivi des vitesses de condensation entre le glyoxal, ci-après désigné G, et l'urée, ci-après désignée U, d'une part, et entre le glyoxal et la N,N'-diméthylurée, ci-après désignée $Dm_eU$, d'autre part, effectuées soit sans catalyseur, soit en présence de substances tampons telles que l'acide citrique et ses sels, l'acétate de sodium, soit enfin en présence d'acide orthophosphorique ont permis de mettre en évidence un double avantage d'une catalyse à l'acide orthophosphorique, selon l'invention, à savoir : une accélération de la vitesse de la réaction de condensation attendue et deuxièmement, un ralentissement de la vitesse de la condensation parasite de l'urée cyclique formée de formule générale (II) dans laquelle R représente un atome d'hydrogène avec du glyoxal non transformé.

Ces vitesses réactionnelles ont été déterminées, sur des prises d'essais prélevées à intervalles réguliers dans les milieux réactionnels, soit par analyse thermique différentielle, ATD, soit par chromatographie liquide à haute pression, HPLC, soit par dosage chimique du glyoxal libre.

On a ainsi mis en évidence les faits expérimentaux suivants mentionnés dans le tableau ci-après et recueillis, d'une part, dans la condensation d'une mole de glyoxal en solution aqueuse à 40 % en poids avec une mole d'urée, effectuée à pH = 6 et à 40 °C soit sans catalyseur, essai désigné a, soit en présence de 7,14 mmoles d'acide citrique monohydraté et de 48,8 mmoles d'acétate de sodium, essai désigné b, soit enfin en présence de 28,5 mmoles d'acide orthophosphorique en solution à 85 % en poids dans l'eau ; d'autre part, dans la condensation d'une mole de glyoxal en solution aqueuse à 40 % en poids avec 1,07 mole de N,N'-diméthylurée, effectuée à pH = 6 et à 40 °C soit sans catalyseur, essai d, soit en présence de 4,7 mmoles d'acide citrique monohydraté et de 39 mmoles d'acétate de sodium, essai e, soit enfin en présence de 28,5 mmoles d'acide orthophosphorique en solution aqueuse à 85 % en poids, essai f.

Tableau

| | DHEU | | | $DM_eDHEU$ | | |
|---|---|---|---|---|---|---|
| | essai a | essai b | essai c | essai d | essai e | essai f |
| $\Delta H$ kcal/mole | 4,9 | 4,5 | 5,1 | 8,0 | 9,5 | 12,7 |

Tableau (Suite)

| | DHEU | | | DM$_e$DHEU | | |
|---|---|---|---|---|---|---|
| | essai a | essai b | essai c | essai d | essai e | essai f |
| EA kcal/mole | 19,1 | 23,2 | 19,8 | 25,6 | 14,8 | 15,5 |
| $\log_e k$ $s^{-1}$ | 23 | 30 | 26 | 30 | 16 | 18 |
| constante de vitesse k $l.mole^{-1}min^{-1}$ | 0,0015 | 0,0025 | 0,0097 | 0,00029 | 0,0076 | 0,01 |
| vitesse relative | 1 | 1,7 | 6,5 | 1 | 26 | 34 |

On constate donc, à l'analyse de ce tableau, une nette accélération de la vitesse de condensation du glyoxal aved, soit l'urée, soit la N,N'-diméthylurée, lorsque celle-ci est effectuée selon l'invention en présence d'acide orthophosphorique et que cet effet catalytique est encore significatif lorsque cette condensation est réalisée en présence de substances tampons telle que le couple acide citrique-acétate de sodium.

Par ailleurs, on a déterminé l'influence de l'acide orthophosphorique sur la vitesse de la condensation secondaire, parasite, du glyoxal sur la DHEU qui est l'une des réactions secondaires redoutées. Pour ce faire, on a fait réagir à pH = 6 et à 40 °C, une mole de glyoxal en solution aqueuse à 40 % en poids avec une mole de DHEU, soit sans catalyseur, essai g, soit en présence de 60 mmoles d'acide orthophosphorique en solution aqueuse à 85 % en poids, essai h. Selon l'essai g, la constante de vitesse est de 6,1 $10^{-2}$ moles · $l^{-1}$ · $min^{-1}$ et selon l'essai h, elle est de 1,2$10^{-2}$ moles · $l^{-1}$ · $min^{-1}$, soit 5 fois moins rapide.

On remarque également qu'en l'absence d'acide orthophosphorique, cette réaction secondaire est environ 40 fois plus rapide que la réaction principale glyoxal sur urée alors qu'en présence de quantité catalytique d'acide orthophosphorique elle n'est que de 1,2 fois plus rapide. On a donc mis ainsi en évidence le double avantage de la catalyse à l'acide orthophosphorique mentionné précédemment.

L'expérience montre également que la vitesse de condensation du glyoxal avec l'urée de formule générale (I) est d'autant plus rapide que la température du milieu réactionnel est plus élevée. Toutefois, au-dessus de 60 °C, plus la température s'élève, plus le milieu réactionnel se colore, ce qui entraîne au niveau de l'urée cyclique de formule générale (II) formée, l'apparition de colorations parasites et un rendement stationnaire ou même parfois plus faible que celui obtenu à une température inférieure à 60 °C.

Comme attendu, on constate aussi qu'en effectuant le procédé selon l'invention avec un excès d'urée de formule générale (I) par rapport au glyoxal engagé, on favorise simultanément la consommation de glyoxal et le rendement en urée cyclique cherchée. Toutefois, un excès trop important d'urée libre dans le milieu réactionnel est nuisible, d'une part, à la cristallisation l'urée cyclique cherchée, et, d'autre part, à l'emploi du milieu réactionnel brut à la préparation d'apprêts textiles.

En conséquence, et ainsi qu'il est connu par ailleurs, le procédé selon la présente invention est réalisé avec un rapport molaire glyoxal sur urée de formule générale (I) compris entre 0,6 et 1. Selon des conditions préférentielles, ce rapport molaire glyoxal sur urée de formule générale (I) est de 0,75 lorsque R représente un atome d'hydrogène et de 0,935 lorsque R représente un radical méthyle.

Dans ce type de condensation, l'influence du pH est également connue et importante. Un pH inférieur à 4 favorise la bicondensation de l'urée de formule générale (I) avec le glyoxal pour conduire à des structures bicycliques de type glycolurile et un pH supérieur à 7 conduit à des urées cycliques de formule générale (II) colorées ainsi qu'à une dégradation du glyoxal par réaction de Cannizzaro.

Le procédé selon l'invention est donc mis en œuvre à un pH compris entre 4 et 7 et préférentiellement à un pH compris entre 5,8 et 7,0.

Les urées cycliques de formule générale (II) sont solubles dans l'eau : la DHEU présente une solubilité dans l'eau à 20 °C de 28 g dans 100 g d'eau et la DM$_e$DHEU une solubilité dans les mêmes conditions de 64 g dans 100 g d'eau. Quant aux réactifs : le glyoxal et l'acide orthophosphorique ils sont

commercialisés en solution aqueuse, le premier habituellement à 40 % en poids et le second communément à 85 % en poids. Les urées de formule générale (I) sont aussi solubles dans l'eau. En conséquence, le procédé selon la présente invention effectué en milieu aqueux est mis en œuvre au départ de ces réactifs commerciaux sans introduction supplémentaire d'eau et en fin de réaction, si l'on désire isoler à l'état cristallisé l'urée cyclique de formule générale (II) cherchée, il est généralement nécessaire d'éliminer sous vide à une température inférieure à 50 °C une partie de l'eau apportée par la solution aqueuse de glyoxal utilisée.

Soit à l'état cristallisé, soit à l'état brut c'est-à-dire en solution dans leur milieu de préparation, les urées cycliques de formule générale (II) obtenues selon le procédé de l'invention peuvent être utilisées, entre autres, pour l'obtention d'apprêts textiles ainsi qu'il est décrit dans le brevet US-A-3 260 565. En particulier, elles peuvent être éthérifiées par un alcanol de formule générale (III)

$$R_1OH \qquad (III)$$

dans laquelle $R_1$ représente un radical alkyle en $C_1$-$C_4$, selon des méthodes connues en soi, pour conduire à des urées cycliques de formule générale (IV)

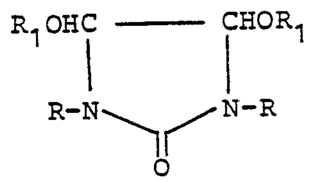

(IV)

dans laquelle R et $R_1$ conservent les significations précitées.

Ces urées cycliques de formule générale (IV) sont des matières premières utilisables à la fabrication d'apprêts textiles.

D'autres avantages et caractéristiques de la présente invention apparaîtront à la lecture de la description suivante et des exemples donnés à titre illustratif et nullement limitatif.

## Exemple 1

On chauffe à 40 °C, sous agitation, à pH = 5,8 la solution suivante obtenue en mélangeant dans l'ordre :

— 678 g (4,67 moles) de glyoxal en solution aqueuse à 40 % en poids,
— 12,6 g (109 mmoles) d'acide orthophosphorique en solution aqueuse à 85 % en poids,
— 15 g (176 mmoles) d'hydroxyde de sodium en solution aqueuse à 47 % en poids,
— 440 g (5 moles) de N,N'-diméthylurée.

A ce stade, selon l'acidité de la solution aqueuse commerciale de glyoxal utilisée, il est parfois nécessaire de moduler la quantité de soude introduite de manière à obtenir le pH de 5,8.

Après 4 heures de chauffage à 40 °C, on obtient une solution limpide présentant une concentration pondérale en glyoxal libre de 0,2 %, soit 40 mmoles et un taux de transformation de 99,1 %. Une analyse chromatographique du milieu réactionnel par HPLC révèle qu'il ne contient, outre de l'eau et des produits minéraux, que de la $DM_eDHEU$ cherchée, 59 % environ, soit 4,63 moles, du glyoxal, 0,2 %, soit 40 mmoles et de la $DM_eU$, 2,84 %, soit 370 mmoles.

Le milieu réactionnel est alors abandonné à la température ambiante pendant 15 heures. La $DM_eDHEU$ cherchée cristallise spontanément. On l'essore, puis on la sèche en étuve ventilée à 50 °C à poids constant.

On isole ainsi un premier jet de 500 g (3,42 moles) de $DM_eDHEU$, présentant un point de fusion de 142 °C.

Les eaux-mères, soit 645 g, sont concentrées à 445 g sous vide à une température inférieure à 50 °C, puis elles sont abandonnées à la cristallisation pendant 15 heures. On isole ainsi un deuxième jet de 95 g (0,65 mole) de $DM_eDHEU$ présentant un point de fusion de 142 °C, sans dépression en mélange avec le premier jet.

Les deux jets sont réunis et on obtient ainsi 595 g (4,07 moles) de dihydroxy-4,5 diméthyl-1,3 imidazolidinone-2, trans, incolore, cristallisée, présentant un point de fusion de 142 °C et un spectre de résonance magnétique nucléaire du proton à 60 MHz en solution dans l'eau lourde en accord avec la structure proposée : 4,9 ppm, s, 2H (CH) ; 2,9 ppm, s, 6H ($N-CH_3$).

Ce produit présente une solubilité à 20 °C de 64 g dans 100 g d'eau. Le rendement global s'établit à 87,2 % de la théorie calculée par rapport au glyoxal mis en œuvre.

## Exemple comparatif 1A

On chauffe à 40 °C, sous agitation, à pH = 5,8 la solution suivante obtenue en mélangeant dans l'ordre :

— 678 g (4,67 moles) de glyoxal en solution aqueuse à 40 % en poids,
— 4,62 g (22 mmoles) d'acide citrique monohydraté,
— 15 g (182 mmoles) d'acétate de sodium anhydre,
— 440 g (5 moles) de N,N'-diméthylurée.

A ce stade, si nécessaire, on ajuste le pH à 5,8 par addition de soude à 47 % en poids.

Après 4 heures de chauffage à 40 °C, la solution limpide obtenue présente une concentration en glyoxal libre de 2,95 % en poids, soit 579 mmoles et un taux de transformation de 87,8 %.

Le milieu réactionnel est ensuite traité comme dans l'exemple 1 et on isole ainsi 50 g (0,343 mole) de $DM_eDHEU$, incolore cristallisée, présentant un point de fusion de 142 °C, soit un rendement de 7 % de la théorie calculée par rapport au glyoxal mis en œuvre.

## Exemple comparatif 1B

On chauffe à 40 °C, sous agitation, à pH = 5,8 la solution suivante obtenue en mélangeant dans l'ordre :
— 678 g (4,67 moles) de glyoxal en solution aqueuse à 40 % en poids,
— 12,6 g (109 mmoles) d'acide orthophosphorique en solution aqueuse à 85 % en poids,
— 4,62 g (22 mmoles) d'acide citrique monohydraté,
— 15 g (182 mmoles) d'acétate de sodium anhydre,
— 15 g (176 mmoles) d'hydroxyde de sodium en solution aqueuse à 47 % en poids,
— 440 g (5 moles) de N,N'-diméthylurée.

A ce stade, si nécessaire, on ajuste le pH à 5,8 en modulant la quantité de soude introduite.

Après 4 heures de chauffage à 40 °C, la solution limpide obtenue présente une concentration en glyoxal libre de 0,2 % soit 40 mmoles et un rendement de transformation de 99,1 %.

Le milieu réactionnel est ensuite traité comme dans l'exemple 1 et on isole ainsi 300 g (2,05 moles) de $DM_eDHEU$, incolore, cristallisée, présentant un point de fusion de 142 °C, soit un rendement de 44 % de la théorie calculée par rapport au glyoxal mis en œuvre.

## Exemple 2

On chauffe à 50 °C, sous agitation, à pH = 7, une solution obtenue en mélangeant dans l'ordre :
— 145 g (1 mole) de glyoxal en solution aqueuse à 40 % en poids,
— 6,6 g (57 mmoles) d'acide orthophosphorique en solution aqueuse à 85 % en poids,
— 80 g (1,33 mole) d'urée,
— qs d'hydroxyde de sodium en solution aqueuse à 47 % pour obtenir un pH = 7.

Après 2 heures de chauffage à 50 °C, la solution limpide obtenue présente une concentration nulle en glyoxal libre.

Le milieu réactionnel est ensuite abandonné 15 heures à la température ambiante. La DHEU cherchée cristallise spontanément, on l'essore puis on la sèche à poids constant en étuve ventilée à 50 °C.

On isole ainsi un premier jet de 73,2 g (0,62 mole) de DHEU cristallisée, incolore, présentant un point de fusion de 140 °C.

Par concentration des eaux-mères à 120 g sous vide à une température inférieure à 50 °C, on isole un deuxième jet de 12 g (0,1 mole) de DHEU cristallisée, incolore, présentant un point de fusion de 140 °C, sans dépression en mélange avec le premier jet.

Les deux jets sont réunis. On obtient ainsi 85,2 g (0,72 mole) de dihydroxy-4,5 imidazolidinone-2, trans, cristallisée, présentant un point de fusion de 142 °C et un spectre de résonance magnétique nucléaire du proton à 60 MHz dans l'eau lourde en accord avec la structure proposée : 5,1 ppm, s, 2H (CH). Le rendement s'établit à 72 % de la théorie calculée par rapport au glyoxal mis en œuvre.

La DHEU présente une solubilité aqueuse à 20 °C de 28 g dans 100 $cm^3$ d'eau.

## Exemple 3

On chauffe à 40 °C, sous agitation, à pH = 6,0, une solution obtenue en mélangeant dans l'ordre :
— 725 g (5 moles) de glyoxal en solution à 40 % en poids dans l'eau ;
— 33 g (286 mmoles) d'acide orthophosphorique en solution aqueuse à 85 % en poids ;
— 34,5 g (405 mmoles) d'hydroxyde de sodium en solution aqueuse à 47 % en poids ;
— 300 g (5 moles) d'urée.

Si nécessaire, à ce stade, on modifie la quantité de soude introduite de manière à obtenir le pH = 6,0 choisi.

Après 5 heures de chauffage, on obtient une solution limpide présentant une concentration pondérale en glyoxal libre de 2,5 % soit 471 mmoles. On abandonne ensuite le milieu réactionnel à la température ambiante, puis on le traite comme dans l'exemple 2. On isole ainsi 252 g (2,13 moles) de trans DHEU présentant un point de fusion de 142 °C. Le rendement global s'établit à 42,6 % de la théorie calculée par rapport au glyoxal mis en œuvre.

Exemple comparatif 3A

On chauffe à 40 °C, sous agitation, à pH = 6,0 une solution obtenue en mélangeant dans l'ordre :
— 725 g (5 moles) de glyoxal en solution aqueuse à 40 % en poids ;
— 8,2 g (39 mmoles) d'acide citrique monohydraté ;
— 20,1 g (245 mmoles) d'acétate de sodium anhydre ;
— 300 g (5 moles) d'urée.

A ce stade, en fonction de l'acidité de la solution aqueuse commerciale de glyoxal, on ajuste si nécessaire, le pH à 6,0 par addition de soude à 47 % en poids.

Après 5 heures de chauffage, on obtient une solution aqueuse limpide présentant une concentration pondérale en glyoxal libre de 3,3 %, soit 600 mmoles.

On abandonne ensuite le milieu réactionnel à la température ambiante, puis on le traite comme dans l'exemple 3. On isole ainsi 122 g (1,034 mole) de trans DHEU présentant un point de fusion de 142 °C. Le rendement s'établit à 20,7 % de la théorie calculée par rapport au glyoxal mis en œuvre.

**Revendications**

1. Procédé de fabrication d'urées cycliques de formule générale (II)

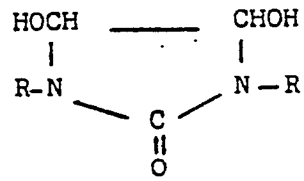

(II)

dans laquelle R représente un atome d'hydrogène ou un radical alkyle en $C_1$-$C_4$ par condensation en milieu aqueux, à une température comprise entre 40 °C et 60 °C, de glyoxal avec un excès ou non, d'une urée de formule générale (I)

RNH—CO—NHR

dans laquelle R a la signification ci-dessus, caractérisé par le fait qu'il est réalisé à un pH compris entre 4 et 7 et en présence d'une quantité catalytique d'acide phosphorique.

2. Procédé selon la revendication 1, caractérisé par le fait que la quantité catalytique d'acide orthophosphorique utilisée est comprise entre 2 mmoles et 150 mmoles par mole de glyoxal mis en œuvre.

3. Procédé selon l'une quelconque des revendications 1 et 2, caractérisé par le fait que l'urée de formule générale (I) utilisée est l'urée.

4. Procédé selon l'une quelconque des revendications 1 et 2, caractérisé par le fait que l'urée de formule générale (I) utilisée est la N,N'-diméthylurée.

5. Procédé selon l'une quelconque des revendications 3 et 4, caractérisé par le fait que la quantité catalytique d'acide orthophosphorique utilisée est comprise entre 20 mmoles et 60 mmoles par mole de glyoxal mis en œuvre.

6. Application du procédé selon la revendication 1, dans la préparation d'apprêts textiles.

**Claims**

1. Process for manufacturing cyclic ureas of general formula (II)

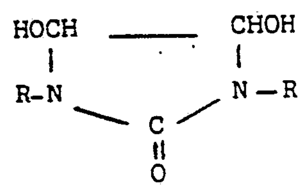

(II)

in which R denotes a hydrogen atom or a $C_1$-$C_4$ alkyl radical, by condensation in aqueous medium, at a temperature of between 40 °C and 60 °C, of glyoxal with an excess or otherwise of a urea of general formula (I)

RNH—CO—NHR

in which R has the above meaning, characterized in that it is carried out at a pH of between 4 and 7 and in the presence of a catalytic quantity of phosphoric acid.

2. Process according to Claim 1, characterized in that the catalytic quantity of orthophosphoric acid used is between 2 millimoles and 150 millimoles per mole of glyoxal employed.

3. Process according to either of Claims 1 and 2, characterized in that the urea of general formula (I) used is urea.

4. Process according to either of Claims 1 and 2, characterized in that the urea of general formula (I) used is N,N'-dimethylurea.

5. Process according to either of Claims 3 and 4, characterized in that the catalytic quantity of orthophosphoric acid used is between 20 millimoles and 60 millimoles per mole of glyoxal employed.

6. Application of the process according to Claim 1, in the preparation of textile finishes.

**Patentansprüche**

1. Verfahren zur Herstellung von cyclischen Harnstoffen der allgemeinen Formel (II)

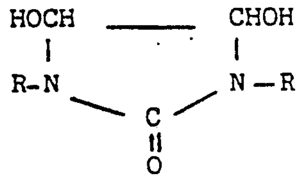

(II)

worin R ein Wasserstoffatom oder einen $C_1$-$C_4$-Alkylrest bedeutet, durch Kondensation in wässrigem Medium bei einer Temperatur zwischen 40 °C und 60 °C von Glyoxal mit oder ohne Überschuß eines Harnstoffs der allgemeinen Formel (I)

RNH—CO—NHR

worin R die zuvor angegebene Bedeutung besitzt, dadurch gekennzeichnet, daß es bei einem pH-Wert zwischen 4 und 7 und in Anwesenheit einer katalytischen Menge von Phosphorsäure durchgeführt wird.

2. Verfahren nach Anspruch 1, dadurch gekennzeichnet, daß die verwendete, katalytische Menge von ortho-Phosphorsäure zwischen 2 mmol und 150 mmol pro mol an eingesetztem Glyoxal liegt.

3. Verfahren nach einem der Ansprüche 1 und 2, dadurch gekennzeichnet, daß der verwendete Harnstoff der allgemeinen Formel (I) Harnstoff ist.

4. Verfahren nach einem der Ansprüche 1 und 2, dadurch gekennzeichnet, daß der verwendete Harnstoff der allgemeinen Formel (I) N,N'-Dimethylharnstoff ist.

5. Verfahren nach einem der Ansprüche 3 und 4, dadurch gekennzeichnet, daß die verwendete, katalytische Menge von ortho-Phosphorsäure zwischen 20 mmol und 60 mmol pro mol an eingesetztem Glyoxal liegt.

6. Anwendung des Verfahrens nach Anspruch 1 bei der Herstellung von Appretierungsmitteln für Textilien.